# EUROPEAN PATENT SPECIFICATION

(11) **EP 2 293 670 B1**
(45) Date of publication and mention of the grant of the patent: **30.03.2016**
(21) Application number: 09758955.0
(22) Date of filing: 15.05.2009
(51) Int. Cl.: A61K 31/555, A01N 37/00, A61P 31/10

(54) **COMPOSITIONS AND METHODS FOR NAIL FUNGUS TREATMENT**
ZUSAMMENSETZUNGEN UND VERFAHREN ZUR BEHANDLUNG VON NAGELBETTPILZEN
COMPOSITIONS ET PROCÉDÉS POUR TRAITEMENT DE MYCOSE D'ONGLE

(30) Priority: 03.06.2008 US 132253
(43) Date of publication of application: 16.03.2011
(73) Proprietor: Arch Chemicals, Inc., NJ 07401 (US)
(72) Inventor: POLSON, George, A., Springboro OH 45066 (US); ROBERTS, Katherine, P., Derby CT 06418 (US); LOU, Khat, Kevin, East Brunswick New Jersey 08816 (US); DINICOLA, Kevin, N., Wolcott CT 06716 (US)
(74) Representative: Riegler, Norbert Hermann
(86) International application number: PCT/US2009/044056
(87) International publication number: WO 2009/148794

(56) References cited:
- WO-A1-87/02580
- WO-A1-2004/000291
- WO-A1-2004/057964
- WO-A1-2004/057964
- US-A- 3 636 213
- US-A- 4 443 222
- US-A- 4 835 149
- US-A1- 2006 165 747
- US-B2- 7 094 422
- GERSTEIN T.: "Clear zinc pyrithione preparations", J. SOC. COSMET. CHEM., vol. 23, 3 February 1972 (1972-02-03), pages 99-114, XP002661089,

## Description

### FIELD OF INVENTION

The present invention relates to topical pyrithione anti-microbial compositions and methods using these compositions for treating microbial infections of the nail.

### BACKGROUND OF THE INVENTION

Fungal infection of the nail, referred to as nail fungus or onychomycosis, is a common affliction that affects approximately 10 to 14% of the population in North America. Globally, it is reported that 1 to 2% of the population suffers from this affliction. This fungal disease of the nail manifests under the nail bed and causes substantial damage to the nail. The symptoms of this disease are a split, thickened, hardened, and rough nail plates, caused by yeast organisms (*Trichophyton mentagrophytes, and Trichophyton rubrum*)*.* Typically, fungal infections are treated by topical application of antifungal agents and/or by oral administration of the drugs.

Due to the potential for side effects which have been associated with some of the oral treatment regimens, it is desirable to treat this disease topically. However, topical treatments of nail fungal disease so far have been problematic. One problem is that the thick nail plate prevents topical antifungal agents from permeating the nail for purposes of reaching the site of the infection. The target sites for the treatment of onychomycosis reside in the nail plate, nail bed and nail matrix. The nail plate is hard, dense, and represents a formidable barrier for drugs to be able to penetrate in a therapeutically required quantity. Although nail material is similar to the stratum corneum of the skin, being derived from epidermis, it is composed primarily of hard keratin, which is highly disulfide-linked, and is approximately 100-fold thicker than stratum corneum. In order to deliver a sufficient amount of drug into the nail plate, the drug active should ideally advantageously be water-soluble to an extent sufficient to render the nail plate permeable by the drug.

Certain nail treating formulations containing various water soluble antifungal agents are known. Illustratively, U.S. patent 6,846,837 discloses the use of an antifungal agent together with a skin-permeation enhancing effective amount of one or more inorganic hydroxides. For the reasons given above, a skin permeation enhancer may not be effective in providing nail permeation enhancement. The '837 patent discloses sodium pyrithione and ciclopirox olamine in a list that includes other antifungal agents. Sodium pyrithione is subject to photodegradation and has a water solubility that is greater than might be desired.

US 2006/165747 discloses a fungicidal intensifier which increases the effectiveness of fungicides and comprises a biocompatible base and an acid salt of a fungicide.

US 3636213 discloses a heavy metal salt solution having fungicidal and bactericidal activity comprising 2-pyridinethione as a stabilising agent.

WO 2004/057964 discloses an antimicrobial composition containing pyrithione, zinc and an organic amine and its use in controlling growth of organisms.

WO 98/02580 discloses a pharmaceutical vehicle for delivering drugs topically to nails, comprising a hydrophilic film-forming resin.

WO 2004/000291 discloses a method and topical pharmaceutical formulation for the treatment of nail fungus capable of permeating the nail having a specific pH.

There is a need in the nail fungus treatment community for another fungicidal compound having greater stability and antifungal efficacy characteristics. The present invention provides one answer to that need.

### OBJECTS OF THE INVENTION

It is an object of the present invention to prepare an effective topical pyrithione anti microbial composition and treatment for fungal infections of the nail.

Another object is to prepare an anti-microbial composition containing solubilized metal pyrithione complexes that can interact with the keratin forming disulfide bonds, thereby passing though the nail bed and surrounding skin in a therapeutically sufficient quantity to eliminate the fungal infection.

A more specific object of this invention is to provide a topical composition for nail fungus comprising a solubilized complex of zinc pyrithione, a penetration enhancer, and a film former. The topical composition provides sustained benefit against nail fungus.

These and other objects will become apparent upon reading the following detailed description of the invention.

### SUMMARY OF THE INVENTION

The present invention provides a formulation and method for the treatment of fungal infections of the nail (onychomycosis) utilizing a topical composition comprising zinc pyrithione, a solubilizer for the zinc pyrithione, a film former, and a volatile solvent. The formulation is preferably in the form of a lacquer. The zinc pyrithione complex may be present in the composition in amounts of from about 0.01 % to about 2%, preferably from 0.01% to about 1%, more preferably about 0.5%, the solubilizer in amounts of from about 1 to about 20%, the film former in amounts of from about 1 to about 20% and the volatile solvent in amounts of from about 1 to 30%, all percentages being by weight of the total composition. Optionally, the topical composition may also include a nail permeation enhancer, such as compounds containing sulfhydryl (SH) groups, terpenes and keratolytic agents, in amounts of from about 1 to about 20 wt %. Optionally, the composition contains additional metal besides that provided by the zinc in the zinc pyrithione in order to enhance the antifungal activity of the pyrithione. The additional metal, if used, is selected from the group consisting of copper, silver, zinc and combinations thereof and is in amounts of from 1 to about 20%.

According to the method or treatment of the invention, the composition containing the metal pyrithione complex is topically applied to the infected nail preferably as a nail polish or lacquer which may be applied with a dropper, swab or the like to the afflicted nail surface. The treatment can be done at least two times a day, with a course of treatment lasting from several weeks to several months or until a cure is effected or a significant reduction of the infection is achieved.

### DETAILED DESCRIPTION OF THE PREFERRED EMBODIMENTS

Zinc pyrithione is a very effective antifungal additive that is useful in treating nail fungus organisms when compared with other topical antifungal drugs. However, the low water solubility of zinc pyrithione limits its ability to permeate nails in order to reach the nail fungus and provide a desired level of bioavailability. The present invention addresses that limitation by providing a composition that contains a solubilizer for the zinc pyrithione in addition to a permeation enhancer in order to improve the nail penetration of the zinc pyrithione as well as its bioavailability at the site of nail fungus situated beneath the nail.

Thus, the compositions of the present invention comprise solubilized metal pyrithione antifungal actives that inhibit the growth of the yeast organisms (Thrichophyton mentagrophytes, and Trichophyton rubrum) that cause nail infections. The present methods and compositions utilize solubilized polyvalent metal salt of pyrithione in a film forming vehicle that when topically applied, can penetrate through the nail to target the fungus responsible for the infection. It has been demonstrated in accordance with the invention that insoluble metal pyrithione complexes can be completely solubilized or complexed and can be made bio-available to the nail fungus, and that formulations can be developed that can penetrate the nail bed and provide relief to afflicted patients. Without wishing to be bound by any particular theory, it is believed that the presence of thio groups in the zinc pyrithione which, when solubilized, are available to form disulfide linkages which help to penetrate the nail though a series of cascading disulfide pathways in view of the disulfide linkages present in the keratin of the nail. Further, the zinc ion that is released when the zinc pyrithione is solubilized serves to potentiate the antifungal activity of the solubilized pyrithione.

Nail lacquer, also known as nail coating, polish, enamel and/or varnish, is useful as a vehicle for delivering zinc pyrithione to the nail in accordance with the present invention since it is convenient and user-friendly. The lacquer formulation containing the zinc pyrithione is desirably non-irritating to the skin and has an acceptable shelf life.

The nail lacquer contains a film former to facilitate the formation of a coating of the lacquer when it is painted onto the nail. Further, in order to enhance nail penetration, a solubilizer for the zinc pyrithione in the nail lacquer is used, optionally together with a nail permeation enhancer. Optionally, elemental metal or metal salt can be added to the lacquer in order to further enhance the effect of the zinc ions contributed by the zinc pyrithione in potentiating the antifungal activity of the pyrithione moiety. To further enhance the effect of the composition, other active ingredients could also be added to the lacquer. The exemplary active ingredients are allylamines (including terbinafine), griseolfulvine, triazoles (including itraconazole and fluconazole), imidazole derivatives (including ketoconazole, miconazole, clotrimazole, and enconazole), amorolfine, bifonazole, hydroxypyriones, ciclopirox olamine, octopirox salts and 2-hydroxy-6-octyl pyridine.

Various film formers, solubilizers, potentiators and nail penetration enhancers are described hereinbelow.

### Solubilizers

U.S. Patent No. 4,835,149, discloses that the insoluble metals salts of pyrithione can be solubilized in common organic solvents and/or water by combination with an amine having the following formula [00017]

HₓN[(CH₂)_{y}X]_{z}

where x is from 0 to about 2, y is from about 1 to 3, z is about 1 to 3, x + z = 3, and X is H, OH or COOH and certain amino carboxylic acids. The addition of alcohol allows for the solubilization of the pyrithione salts using less aliphatic amine and aminocarboxylic acid. The above composition is useful at a pH of about 4.0 to 7.4.

Terry Gerstein discloses that zinc pyrithion is highly soluble in many primary aliphatic amines. (See "Clear Zinc Pyrithione Preparations, J. Soc. Cosmetic. chem. 23, 90-114 (1972)).

It has also been found that nitrogenous bases could be used as solubilizer. Suitable nitrogenous bases may contain any one or a combination of the following:

Primary amino (--NH₂) groups; mono-substituted (secondary) amino groups --NHR where R is hydrocarbyl, generally either alkyl or aryl, e.g., lower alkyl or phenyl, and may be substituted with one or more nonhydrocarbyl substituents, e.g., 1 to 3 halo, hydroxyl, thiol, or lower alkoxy groups (such --NHR groups include, for example, methylamino, ethylamino, isopropylamino, butylamino, cyclopropylamino, cyclohexylamino, n-hexylamino, phenylamino, benzylamino, chloroethylamino, hydroxyethylamino, etc.) di-substituted (tertiary) amino groups --NR^{a} R^{b} where R^{a} and R^{b} may be the same or different and are defined above for R (suitable --NR^{a} R^{b} include, for sample, dimethylamino, diethylamino, diisopropylamino, dibutylamino, methylpropylamino, methylhexylamino, methylcyclohexylamino, ethylcyclopropylamino, ethychloroethylamino, methylbenzylamino, methylphenylamino, methyltoluylamino, methyl-p-chlorophenylamino, methylcyclohexylamino, etc.); amides --(CO)--NR^{c} R^{d} where R^{c} and R^{d} may be the same or different and are either hydrogen or R, wherein R is as defined above (including, for example, amides wherein one of R^{c} and R^{d} is H and the other is methyl, butyl, benzyl, etc.); cyano (--CN); aromatic nitrogen-containing heterocycles, typically five- or six-membered monocyclic substitutents, or bycyclic fused or linked five- or six-membered rings (such as pyrrolyl, pyrrolidinyl, pyridinyl, quinolinyl, indolyl, pyrimidinyl, imidazolyl, 1,2,4-triazolyl,tetrazolyl etc.); and nonaromatic nitrogen-containing heterocycles, typically four- to six-membered rings, including lactams and immides, e.g., pyrrolidino, morpholino, piperazino, piperidino, N-pheyl-propiolactam,butyrolactam, caprolactam,acetimide, phthalimide, succinimide, etc. Primary amines, secondary amines, and tertiary amines may be generically grouped as encompassed by the molecular structure NR¹ R² R³ wherein R¹, R² and R³ are selected from H, alkyl, hydroxyalkyl, alkoxyalkyl, alkenyl, hydroxyalkenyl, alkoxyalkenyl, cycloalkyl, cycloalkyl-substituted alkyl, monocyclic aryl, and monocyclic aryl-substituted alkyl, with the proviso that at least one of R¹, R² and R³ is other than H. Examples of such amines include, diethanolamine, triethanolamine, isopropanolamine, triisopropanolamine, dibutanol amine, tributanol amine, N-dodecylethanolamine, N-(2-methoxyethyl) dodecylamine, N-(2,2-dimethoxyethyl)dodecylamine, N-ethyl-N-(dodecyl)ethanolamine, N-ethyl-N-(2-methoxyethyl)dodecylamine, N-heyl-N-(2,2-dimethoxyethyl)dodecylamine, dimethyldodecylamine-N-oxide, monolauroyl lysine, dipalmitoyl lysine, dodecylamine, stearylamine, phenyltheylamine, triethylamine, PEG-2 oleamine, PEG-5 oleamine, polyethylenimine, dodecyl 2-(N,N-dimethylamino)propionate, bis(2-hydroxytheyl)oleylamine, and combinations thereof. Exemplary primary amines include 2-aminoethanol, 2-aminoheptane, 2-amino-2-amino-2-methyl-1,3 propanediol, 2-amino-2-methyl-1-propanol, n-amylamine, benzylamine, 1,4-butanediamine, n-butylamine, cyclohexylamine, ethylamine, ethylenediamine, methylamine, α-methylbenzylamine, phenethylamine, propylamine, and tris(hydroxymethyl)aminomethane. Examplary secondary amines include compounds that contain groups such as methylamino, ethylamino, isopropylamino, butylamino, cyclopropylamino, cyclohexylamino, n-hexylamino, phenylamino, benzylamino, chloroethylamino, hydroxyethylamino, and so forth. Exemplary secondary amines include diethanolamine, diethylamine, diisopropylamine, and dimethylamine. Exemplary tertiary amines include compounds that contain groups such as dibutylamino, diethylamino, dimethylamino, diisopropylamino, ethylchloroethylamino, ethylcyclopropylamino, methylhexylamino, methylcyclohexylamino, methylpropylamino, methylbenzylamino, methyltoluylamino, and so forth. Exemplary tertiary amines include N,N-diethylaniline, N,N-dimethylglycine, triethanolamine, triethylamine, and trimethlyamine.

### Plasticizers

Plasticizers and non-volatile zinc pyrithione solubilizers may optionally be used in the formulation of the invention. Examples of these substances include, phthalate esters (e.g., dibutyl phthalate), citrate esters, triacetin, isopropyl myristate, N-methyl-2-pyrrolidone, fatty acids and fatty acid esters, propylene glycol, butylene glycol, hexylene glycol, propylene carbonate, poly-propylene glycol, methoxypolyethylene glycol, polyethylene glycol, glycerin. When plasticizers are used, they are preferably about 0.001 to about 10% by weight of the total composition.

### Potentiators

The potentiators used in the composition and treatments of the invention may be any element or compound providing zinc, copper or silver ions. Examples of suitable potentiators include the following: zinc acetate, zinc borate, zinc oxide, zinc carbonate, zinc chloride, zinc sulfate, zinc hydroxide, zinc citrate, zinc fluoride, zinc iodide, zinc lactate, zinc oleate, zinc oxalate, zinc phosphate, zinc propionate, zinc salicylate, zinc selenate, zinc silicate, zinc stearate, zinc sulfide, zinc tannate, zinc tartrate, zinc valerate, basic zinc carbonate, zinc carbonate hydroxide, hydrozincite, zinc copper carbonate hydroxide, aurichalcite, copper zinc carbonate hydroxide, rosasite, phyllosilicate containing zinc iorns, layered double hydroxide, hydroxyl double salts, copper salts such as copper carbonate, cupric hydroxide, silver species such as silver bromide, silver citrate, silver nitrate, silver oxide and mixtures thereof.

### Film Formers

A polymeric film former refers to a polymer which may be added to a volatile solvent and other substances to form a polymeric solution which may be applied to form a film. Examples of polymeric film formers that may be used in the compositions and treatments of the invention include, but are not limited to, acrylic copolymers/acrylic polymers such as CARBOSET^{®} or AVALURE^{®} (both of which are trademarks of B F Goodrich); polymers of methacrylic acid and its esters, such as EUDRAGIT^{®} (which is a trademark of Rohm Pharma): S, L, RS and RL series, cellulose polymers, nitrocellulose, methyl cellulose, ethyl cellulose, cellulose acetates (such as cellulose triacetate, cellulose acetate butyrate); nylon, polyvinyl acetate phthalate, formaldehyde resin, and polymer blends of the aforementioned polymers. Preferred polymeric film formers are selected from the group consisting of acrylic copolymers/acrylic polymers, polymers of methacrylic acid and its esters.

### Volatile Solvents

An example of a volatile solvent includes, but is not limited to, water. Other suitable volatile solvents include ethyl alcohol, isopropyl alcohol, ethyl acetate, butyl acetate, acetone and mixtures thereof.

### Nail Permeation Enhancers

Compounds possessing sulfhydryl (SH) groups, i.e., mercaptan compounds, are known to enhance nail penetration and may be useful for their purpose in composition and treatments for nails. Sulfur-containing cystein derivatives may also be useful in topical preparations for treatment of nail diseases such as onychomycosis. U.S. Pat. No. 5,696,164 to Sun et al. discloses the use of sulfhydryl-containing cysteine and N-acetyl cysteine in combination with urea to increase drug permeability in a nail plate. U.S. Pat. No. 6,123,930 provides a composition of sulphur-bearing amino acid together with sodium tetraborate for the treatment of nails.

Terpenes are also known to be effective skin penetration enhancers. Menthone, in particular, has been found to enhance penetration of several different drugs across skin. Dithiothreitol, which contains two SH groups, has been shown to be a particularly effective reducing agent and may be used.

Keratolytic agents such as Salicylic acid (SA), urea (U) and guanidine hydrochloride (GnHCI) are substances which may disrupt the tertiary structure, and possibly secondary linkages (such as hydrogen bonds) in keratin, thus promoting penetration through the nail. Preferred penetration enhancers are urea and compounds containing urea groups.

### Experimental

Several water based and waterless solutions containing the pyrithione metal complex, a solubilizer, film former and a volatile solvent have been made and studied for both efficacy against the nail fungus organisms as well as for stability of the formulations.

These formulations were found to be stable after storage at temperatures of 4°C and 45°C over a period of at least four weeks duration.

### Microbiology Efficacy Test

The following formulations were used in the tests:

Formulation A (10-01/1) contained approximately 0.5% zinc pyrithione complex (ZPT) and was anhydrous.

Formulation B (10-01/2) contained approximately 0.75% ZPT and was also anhydrous.

Formulation C (10-01/10) contained approximately 1.0% ZPT and was also anhydrous.

Formulation D (10-2/4) contained approximately 1.0% ZPT and was an aqueous solution.

Formulation E (10-7/1) contained approximately 1.0% sodium pyrithione complex (NaPT) and was anhydrous.

Each test formulation (50 microliters) was pipetted onto sterile ½ inch paper disks and was allowed to air dry. Spore suspensions of Trichophyton mentagrophytes and Trichophyton rubrum, were made using standard microbiological techniques. The resulting suspensions contained approximately 10,000,000 spores per milliliter. Sterile swabs were used to distribute the spore suspensions over the surface of Potato Dextrose Agar plates and one sample disk was placed in the center of each plate. The average results of these tests are shown in Table 1.

**Table 1.**

| Formulations Applied to Paper Disks - Zone of Inhibition (mm) | | |
|---|---|---|
| Topical Formulation | T. mentagrophytes | T. rubrum |
| Formulation A-(10-01/1) (0.5% ZPT) | 19.75 | ≥33.5 |
| Formulation B-(10-01/2) (0.75% ZPT) | 25.0 | 30.5 |
| Formulation C-(10-01/10) (1.0% ZPT) | 28.75 | ≥36 |
| Formulation D-(10-2/4) (1.0% ZPT) | 29.62 | ≥36 |
| Formulation E-(10-7/1) (1.0% NaPT) | 27.50 | ≥36 |
| Comparative Example using Ciclopirox | 16.0 | 19.0 |
| Control - No pyrithione - aqueous base | No zone | No zone |

It will be seen from Table 1 that the Zone of Inhibition was greatest (29.75 mm) in the case of Formulation D containing the largest percentage (1.0 %) of the pyrithione salt (ZPT) . Formulation A containing the least amount of the ZPT (0.5%) showed the smallest Zone of Inhibition (18.5 mm) which nevertheless was larger than that of the control formulation.

As an additional test, a synthetic nail substrate, IMS VITRO-NAILS, was used to simulate permeation of the active through a human nail. This material is reported to have the wetting properties, thickness and flexibility of human nails. This experiment was conducted in the same manner as first experiment above, except that the formulations were pipetted onto ¾-1 inch squares of VITRO-NAILS and either allowed to dry for 2 days before testing or place directly on the inoculated agar plates while wet. The results of tests are shown in Table 2.

**Table 2.**

| Formulations Applied to Artificial Nail - T. mentagrophytes Zone of Inhibition (mm) | | |
|---|---|---|
| Formulation | Tested Dry | Tested Wet |
| Formulation A-(10-01/1) (0.5% ZPT) | 16 | 29 |
| Formulation B-(10-01/2) (0.75% ZPT) | 14.5 | 22.5 |
| Formulation C-(10-01/10) (1.0% ZPT) | 26.75 | 22.5 |
| Formulation D-(10-2/4) (1.0% ZPT) | 25 | 29.25 |
| Formulation E-(10-7/1) (NaPT) | 15 | 20.5 |
| Comparative Example using Ciclopirox | 8.75 | 9 |
| No treatment | 5.5 | Not done |

It will be seen from Table 2 that the nail treatment formulation of the invention showed the greater effect in inhibiting the fungus growth as compared to the control over the entire range of ZPT concentrations. The Zone of Inhibition was the greatest (26.75 mm) in this case for the Formulation D again containing the largest percentage (1.0 %) of the pyrithione salt (ZPT) . Formulation A containing the least amount of the ZPT (0.5%) showed the smallest Zone of Inhibition (16 mm) which was nearly twice that of the control formulation. The control formulation was only slightly better in inhibiting growth than the untreated artificial nail sample.

## Claims

1. A composition for the topical treatment of nail fungus comprising zinc pyrithione, a solubilizer for the zinc pyrithione, a film former, and a volatile solvent, wherein the metal pyrithione is present in amounts of from about 0.01 to about 2.0 wt. %, the solubilizer is present in amounts of from about 1.0 to about 20 wt. %, the film former is present in amounts of from about 1.0 to about 20 wt. % and the volatile solvent is present in amounts of from about 1.0 to about 30 wt. %.

2. A composition according to claim 1 further including a potentiator selected from a zinc source, a copper source, a silver source, and combinations thereof in order to enhance the efficacy of the pyrithione present in the composition, the potentiator present in amounts of from about 1.0 to about 20 wt. %.

3. A composition according to claim 2, wherein the zinc source is selected from the group consisting of zinc acetate, zinc borate, zinc oxide, zinc carbonate, zinc chloride, zinc sulfate, zinc hydroxide, zinc citrate, zinc fluoride, zinc iodide, zinc lactate, zinc oleate, zinc oxalate, zinc phosphate, zinc propionate, zinc salicylate, zinc selenate, zinc silicate, zinc stearate, zinc sulfide, zinc tannate, zinc tartrate, zinc valerate, basic zinc carbonate, zinc carbonate hydroxide, hydrozincite, zinc copper carbonate hydroxide, aurichalcite, copper zinc carbonate hydroxide, rosasite, phyllosilicate containing zinc ions, layered double hydroxide, hydroxyl double salts and combinations thereof.

4. A composition according to claim 1, further including a nail permeation enhancer in amounts of from about 1.0 to about 20 wt. % of the composition.

5. A composition according to claim 4 wherein the permeation enhancer is urea or a urea group-containing compound.

6. A composition according to claim 1, wherein the solubilizer is an organic solvent or water in combination with an amine having the formula
HₓN[(CH₂)_{y}X]_{z}
where x is from 0 to about 2, y is from about 1 to 3, z is from about 1 to 3, x + z = 3, and where X is H, OH or COOH and an amino carboxylic acid.

7. A composition according to claim 5, further including an alcohol.

8. A composition according to claim 6, wherein the pH is from about 4.0 to about 7.4.

9. A composition according to claim 1, wherein the solubilizer is a nitrogenous base selected from primary amino groups, secondary amino groups, aromatic nitrogen-containing heterocycles, non-aromatic nitrogen containing heterocycles, and amines.

10. A composition according to claim 1, wherein the solubilizer is selected from n-dodecylamine, 1,2-aminopropane, ethanolamine, diglycol amine, diethanolamine, triethanolamine, diisopropanolamine, triisopropanolamine, mixed isopropanolamines, 2-amino-2-methyl-1-propanol (also called AMP), 2-amino-2-ethyl-1,3-propanediol (also called AEPD), 2-(2-aminoethoxy)ethanol (also called diglycol amine), N-methyldiethanolamine, N,N-dimethylethanolamine, N,N-diethylethanolamine, N,N-dibutylaminoethanol, N,N-dimethylamino-2-propanol, and combinations thereof.

11. A composition according to claim 1, wherein the nitrogenous base is polyethylenimine.

12. A composition according to claim 1, wherein the film former is selected from the group of acrylic copolymers, acrylic polymers, polymers of methacrylic acid and its esters, cellulose polymers, nitrocellulose, methyl cellulose, ethyl cellulose, cellulose acetate, nylon, polyvinyl acetate phthalate, formaldehyde resin, and polymer blends of the aforementioned polymers.

13. A composition according to claim 1, wherein the volatile solvent is selected from a group consisting of ethyl alcohol, isopropyl alcohol, ethyl acetate, butyl acetate, acetone and mixtures thereof.

14. A composition according to claim 1 further including an active ingredient selected from the group of allylamines, griseofulvine, triazoles, imidazole derivatives, amorolfine, bifonazole, hydroxypyridones, ciclopirox olamine, octopirox salts and 2-hydroxy-6-octylpyridine and mixtures thereof.

15. A composition according to claim 1 for use in treating the human nail against fungus infection.

## Patentansprüche

1. Zusammensetzung zur topischen Behandlung von Nagelpilz, beinhaltend Zinkpyrithion, einen Lösungsvermittler für Zinkpyrithion, einen Filmbildner und ein flüchtiges Lösungsmittel, wobei das Metallpyrithion in Mengen von etwa 0,01 bis etwa 2,0 Gew.-% vorliegt, der Lösungsvermittler in Mengen von etwa 1,0 bis etwa 20 Gew.-% vorliegt, der Filmbildner in Mengen von etwa 1,0 bis etwa 20 Gew.-% vorliegt und das flüchtige Lösungsmittel in Mengen von etwa 1,0 bis etwa 30 Gew.-% vorliegt.

2. Zusammensetzung nach Anspruch 1, ferner enthaltend einen Potentiator, ausgewählt aus einer Zinkquelle, einer Kupferquelle, einer Silberquelle und Kombinationen davon, zur Verstärkung der Wirksamkeit des in der Zusammensetzung vorliegenden Pyrithions, wobei der Potentiator in Mengen von etwa 1,0 bis etwa 20 Gew.-% vorliegt.

3. Zusammensetzung nach Anspruch 2, wobei die Zinkquelle aus der Gruppe bestehend aus Zinkacetat, Zinkborat, Zinkoxid, Zinkcarbonat, Zinkchlorid, Zinksulfat, Zinkhydroxid, Zinkcitrat, Zinkfluorid, Zinkiodid, Zinklactat, Zinkoleat, Zinkoxalat, Zinkphosphat, Zinkpropionat, Zinksalicylat, Zinkselenat, Zinksilikat, Zinkstearat, Zinksulfid, Zinktannat, Zinktartrat, Zinkvalerat, basischem Zinkcarbonat, Zinkcarbonathydroxid, Hydrozinkit, Zinkkupfercarbonathydroxid, Aurichalcit, Kupferzinkcarbonathydroxid, Rosasit, Zinkionen enthaltendem Phyllosilikat, schichtartigem Doppelhydroxid, Hydroxyldoppelsalz und Kombination davon ausgewählt ist.

4. Zusammensetzung nach Anspruch 1, ferner enthaltend einen Nagelpermeationsverbesserer in Mengen von etwa 1,0 bis etwa 20 Gew.-% der Zusammensetzung.

5. Zusammensetzung nach Anspruch 4, wobei es sich bei dem Permeationsverbesserer um Harnstoff oder um eine harnstoffhaltige Verbindung handelt.

6. Zusammensetzung nach Anspruch 1, wobei es sich bei dem Lösungsvermittler um ein organisches Lösungsmittel oder Wasser in Kombination mit einem Amin der Formel
HₓN[(CH₂)_{y}X]_{z}
wobei x für 0 bis etwa 2 steht, Y für etwa 1 bis 3 steht, z für etwa 1 bis 3 steht, x + z = 3 und wobei X für H, OH oder COOH und eine Aminocarbonsäure steht.

7. Zusammensetzung nach Anspruch 5, ferner enthaltend einen Alkohol.

8. Zusammensetzung nach Anspruch 6, wobei der pH-Wert etwa 4,0 bis etwa 7,4 beträgt.

9. Zusammensetzung nach Anspruch 1, wobei es sich bei dem Lösungsvermittler um eine stickstoffhaltige Base handelt, die aus primären Aminogruppen, sekundären Aminogruppen, aromatischen Stickstoff enthaltenden Heterocyclen, nicht-aromatischen Stickstoff enthaltenden Heterocyclen und Aminen ausgewählt ist.

10. Zusammensetzung nach Anspruch 1, wobei der Lösungsvermittler aus n-Dodecylamin, 1,2-Aminopropan, Ethanolamin, Diglykolamin, Diethanolamin, Triethanolamin, Diisopropanolamin, Triisopropanolamin, gemischten Isopropanolaminen, 2-Amino-2-methyl-1-propanol (das auch als AMP bezeichnet wird), 2-Amino-2-ethyl-1,3-propandiol (das auch als AEPD bezeichnet wird), 2-(2-Aminoethoxy)ethanol (das auch als Diglykolamin bezeichnet wird), N-Methyldiethanolamin, *N,N-*Dimethylethanolamin, N,N-Diethylethanolamin, *N,N-*Dibutylaminoethanol, N,N-Dimethylamino-2-propanol und Kombinationen davon ausgewählt ist.

11. Zusammensetzung nach Anspruch 1, wobei es sich bei der stickstoffhaltigen Base um Polyethylenimin handelt.

12. Zusammensetzung nach Anspruch 1, wobei der Filmbildner aus der Gruppe bestehend aus Acrylcopolymeren, Acrylpolymeren, Polymeren von Methacrylsäure und Methacrylsäureestern, Cellulosepolymeren, Nitrocellulose, Methylcellulose, Ethylcellulose, Celluloseacetat, Nylon, Polyvinylacetatphthalat, Formaldehydharz und Polymermischungen der oben aufgeführten Polymere ausgewählt ist.

13. Zusammensetzung nach Anspruch 1, wobei das flüchtige Lösungsmittel aus der Gruppe bestehend aus Ethylalkohol, Isopropylalkohol, Essigsäureethylester, Essigsäurebutylester, Aceton und Mischungen davon ausgewählt ist.

14. Zusammensetzung nach Anspruch 1, ferner enthaltend einen Wirkstoff aus der Gruppe bestehend aus Allylaminen, Griseofulvin, Triazolen, Imidazolderivaten, Amorolfin, Bifonazol, Hydroxypyridonen, Ciclopirox Olamine, Octopiroxsalzen und 2-Hydroxy-6-octylpyridin und Mischungen davon.

15. Zusammensetzung nach Anspruch 1 zur Verwendung bei der Behandlung des menschlichen Nagels gegen Pilzinfektion.

## Revendications

1. Composition destinée au traitement topique d'une mycose des ongles, comprenant de la pyrithione de zinc, un solubilisant pour la pyrithione de zinc, un agent filmogène, et un solvant volatil, **caractérisée en ce que** la pyrithione de métal est présente selon des quantités allant d'environ 0,01 à environ 2,0% en poids, le solubilisant est présent selon des quantités allant d'environ 1,0 à environ 20% en poids, l'agent filmogène est présent selon des quantités allant d'environ 1,0 à environ 20% en poids, et le solvant volatil est présent selon des quantités allant d'environ 1,0 à environ 30% en poids.

2. Composition selon la revendication 1, comprenant en outre un potentialisateur choisi parmi une source de zinc, une source de cuivre, une source d'argent, et des combinaisons de celles-ci, afin d'accroître l'efficacité de la pyrithione présente dans la composition, le potentialisateur étant présent selon des quantités allant d'environ 1,0 à environ 20% en poids.

3. Composition selon la revendication 2, **caractérisée en ce que** la source de zinc est choisie dans le groupe constitué par l'acétate de zinc, le borate de zinc, l'oxyde de zinc, le carbonate de zinc, le chlorure de zinc, le sulfate de zinc, l'hydroxyde de zinc, le citrate de zinc, le fluorure de zinc, l'iodure de zinc, le lactate de zinc, l'oléate de zinc, l'oxalate de zinc, le phosphate de zinc, le propionate de zinc, le salicylate de zinc, le sélénate de zinc, le silicate de zinc, le stéarate de zinc, le sulfure de zinc, le tannate de zinc, le tartrate de zinc, le valérate de zinc, le carbonate de zinc basique, l'hydroxycarbonate de zinc, l'hydrozincite, l'hydroxycarbonate de zinc et de cuivre, l'aurichalcite, l'hydroxycarbonate de cuivre et de zinc, la rosasite, un phyllosilicate contenant des ions zinc, l'hydroxyde double lamellaire, le sel double d'hydroxyle, et des combinaisons de ceux-ci.

4. Composition selon la revendication 1, comprenant en outre un améliorateur de la perméation des ongles selon des quantités allant d'environ 1,0 à environ 20% en poids de la composition.

5. Composition selon la revendication 4, **caractérisée en ce que** l'améliorateur de la perméation est l'urée ou un composé contenant des groupements urée.

6. Composition selon la revendication 1, **caractérisée en ce que** le solubilisant est un solvant organique ou de l'eau en combinaison avec une amine répondant à la formule
HₓN[(CH₂)_{y}X]_{z}
où x va de 0 à environ 2, y va d'environ 1 à 3, z va d'environ 1 à 3, x + z = 3, et où X est H, OH ou COOH et un acide aminocarboxylique.

7. Composition selon la revendication 5, comprenant en outre un alcool.

8. Composition selon la revendication 6, **caractérisée en ce que** le pH va d'environ 4,0 à environ 7,4.

9. Composition selon la revendication 1, **caractérisée en ce que** le solubilisant est une base azotée choisie parmi les groupements amine primaire, les groupements amine secondaire, les hétérocycles azotés aromatiques, les hétérocycles azotés non aromatiques, et les amines.

10. Composition selon la revendication 1, **caractérisée en ce que** le solubilisant est choisi parmi la n-dodécylamine, le 1,2-aminopropane, l'éthanolamine, la diglycol amine, la diéthanolamine, la triéthanolamine, la diisopropanolamine, la triisopropanolamine, les isopropanolamines mixtes, le 2-amino-2-méthyl-1-propanol (également appelé AMP), le 2-amino-2-éthyl-1,3-propanediol (également appelé AEPD), le 2-2-aminoéthoxy)éthanol (également appelé diglycol amine), la N-méthyldiéthanolamine, la N,N-diméthyléthanolamine, la N,N-diéthyléthanolamine, le N,N-dibutylaminoéthanol, le N,N-diméthylamino-2-propanol, et des combinaisons de ceux-ci.

11. Composition selon la revendication 1, **caractérisée en ce que** la base azotée est la polyéthylèneimine.

12. Composition selon la revendication 1, **caractérisée en ce que** l'agent filmogène est choisi dans le groupe constitué par les copolymères acryliques, les polymères acryliques, les polymères d'acide méthacrylique et ses esters, les polymères de cellulose, la nitrocellulose, la méthylcellulose, l'éthylcellulose, l'acétate de cellulose, le nylon, l'acétate-phtalate de polyvinyle, une résine formaldéhyde, et des mélanges de polymères issus des polymères susmentionnés.

13. Composition selon la revendication 1, **caractérisée en ce que** le solvant volatil est choisi dans le groupe constitué par l'alcool éthylique, l'alcool isopropylique, l'acétate d'éthyle, l'acétate de butyle, l'acétone et des mélanges de ceux-ci.

14. Composition selon la revendication 1, comprenant en outre un ingrédient actif choisi dans le groupe constitué par les allylamines, la griséofulvine, les triazoles, les dérivés d'imidazole, l'amorolfine, le bifonazole, les hydroxypyridones, le ciclopirox olamine, les sels d'octopirox et la 2-hydroxy-6-octylpyridine, et des mélanges de ceux-ci.

15. Composition selon la revendication 1, pour une utilisation dans le traitement des ongles humains contre une infection fongique.
